# EUROPEAN PATENT APPLICATION

(11) **EP 2 161 009 A1**
(43) Date of publication of application: **10.03.2010**
(21) Application number: 08764364.9
(22) Date of filing: 20.05.2008
(51) Int. Cl.: A61F 2/82, A61F 2/06

(54) **STENT GRAFT INDWELLING DEVICE, STENT GRAFT, AND FIXING TIP**

(30) Priority: 21.05.2007 JP 2007134517
(71) Applicant: Kawasumi Laboratories, Inc., Tokyo 140-8555 (JP); Yokoi, Yoshihiko, Tokyo 168-0072 (JP)
(72) Inventor: YOKOI, Yoshihiko, Tokyo 168-0072 (JP); AKITA, Kazumi, Bungo-Ono-shi Oita 879-7153 (JP)
(74) Representative: Hering, Hartmut
(86) International application number: PCT/JP2008/059204
(87) International publication number: WO 2008/143243

(57) **Abstract**

Provided are a stent graft indwelling device, which facilitates the adjustment of release or indwelling of a stent graft from or in a blood vessel by attaching a plurality of holding rings of wire when the stent graft is caused to indwell in the blood vessel, a stent graft and a fixed chip. The stent graft hence comprises adjusting means for adjusting the insertion angle and/or indwelling position of a stent graft (60) when the stent graft (6) is released from a sheath (30) and caused to be indwell, and the adjusting means for adjusting the insertion angle and/or indwelling position of a stent graft (60) includes a fixed chip (20) containing a contrast medium and/or a holding ring (69A) attached to a rearmost tubular unit (61) of the stent graft (60).

## Description

### Technical Field

This invention relates to a stent graft indwelling device that is designed for reliably indwelling a stent graft at a safe site of a diseased part for the treatment of an ecstatic disease (aneurysm), an arterial constrictive diseases or some other diseases and that decreases the pains and burdens of a patient and reduces the cost or expenses, and relates also to improvements in a fixed chip to be loaded on a dilator of the stent graft indwelling device and a stent graft.

### Background Art

Conventionally, there is known a stent graft indwelling device having a catheter, a pushing rod (also called "dilator") and a sheath, in which a swollen portion that has the form, for example, of an ellipsoid, for blocking or sealing a leading end of the sheath is provided in the vicinity of the leading end of the catheter and the swollen portion is provided with an engagement means such as a notch or the like so that a hook attached to the leading end of a stent unit can be removably engaged therewith (for example, see Patent Document 1).

Further, there is proposed a stent graft indwelling device having a dilator composed of a base body portion, a stent graft holding portion and a head portion and a sheath loadable with the dilator, in which the head portion is shaped in the form of a semi-ellipsoid or a semi-sphere and is partly projected from the leading end of the sheath for blocking or sealing the leading end of the sheath, that part of the head portion inside the sheath is provided with an engagement means such as a notch or the like such that a hook attached to the stent is removably engaged therewith and one or a plurality of contrast medium delivery channel(s) and a guide wire guiding channel are provided in the above dilator (for example, see Patent Document 2).

The above proposed stent graft indwelling devices have the following technical features.
That is, (a) it is ensured that safe and reliable indwelling of the stent graft is made even in an intracorporeal tubular organ such as an extremely curved blood vessel, etc., and pains and burdens of a patient can be reduced. (b) The entering of a body fluid such as arterial blood, etc., into a stent graft portion held in the sheath can be prevented, and the stent graft held in the sheath can be safely delivered into an intracorporeal tubular member such as a blood vessel (artery) without damaging the inner wall thereof.

(c) The catheter is inserted into an intracorporeal tubular member such as blood vessel while a contrast medium or imaging agent is ejected from a contrast medium ejecting port, whereby the insertion site of the catheter can be monitored by image, and the catheter is moved forward and backward while the contrast medium is ejected from the contrast medium ejecting port, thereby allowing an imagewise spotting of a safety site in a diseased part of the intracorporeal tubular member such as a blood vessel in a short period of time. Therefore, the insertion of the stent graft housed in the sheath into the intracorporeal tubular member such as a blood vessel and the determining of indwelling position of the stent graft are made safe and easy.

(d) When the stent graft is pushed out of the sheath and self-dilated in a pre-determined indwelling or placement position in an intracorporeal tubular member such as a blood vessel, the undesired shifting of the stent graft caused by the pressure of a body fluid such as a blood flow is prevented.
(e) Broadened application of the stent graft can be made to the treatment of aneurysm, etc., to which no conventional stent grafts have ever been applied, thereby a beneficial treatment method for many patients with aneurysm, etc., can be provided. And, (f) there can be produced advantageous effects that the direction of the curvature-based stent graft design methodology can be ensured and the like.
   These stent graft indwelling devices that have been already proposed above have the above excellent features and produce remarkable advantageous effects as compared with other conventional devices.

However, unfortunately, these stent graft indwelling devices have a so-called one-piece structure in which the dilator is formed integrally with its head portion (called a swollen portion or fixed chip portion as well) of the dilator. Thereafter, the present inventors later found it desirable to ensure that the stent graft does not rotate in the sheath, that it does not move forward or backward and that it does not come off in the sheath. Further, it was found that when the stent graft is to be released from the sheath, it is desirable that the stent graft should come off easily without any resistance to release. However, the structure of the above-described dilator head has made it difficult to overcome these problems which are desired to overcome.

Further, the dilator has the head portion whose swollen portion has a form greatly expanded like a spindle as compared with a holding portion (for example, see Fig. 2 of JP 2000-350785 A), so that it has not been easy to process a metal mold for integrally molding the dilator with an injection machine or the like. In actual medical care facilities, therefore, a craft man produces each dilator with swollen portion piece by piece cutting a work, followed by thermal processing, etc., and the production thereof hence takes much time. It has also been found those products show variation in form.

The following problems have been also found. In the above stent graft indwelling device, the form of the above swollen portion of the dilator is fixed, so that the sheath cannot always be inserted at a constant angle to the form of a blood vessel (i.e., in conformity with the form of a blood vessel) when inserted. It has been also found that since the stent graft is released into a blood vessel while it is dilated unit by unit, it is difficult to finely or precisely adjust the exact position and fixing angle.

Further, the blood vessel size and the degree of curvature thereof differ depending upon individual patients, and when these stent graft indwelling devices are used for a patient having an extremely or excessively curved blood vessel or for a patient having a boss in the distal arc portion of a thoracic aorta, it has not been necessarily easy to safely place the released stent graft.

That is because, when the stent graft is to be placed in the center (heart) side rather than in a nutrient canal branch portion of a captu-cervical part in the arc portion of an aorta, it is the most important objective to make sure that the contact of the stent graft base body and the leading end of the sheath to the greater curvature side of the arc portion of the thoracic aorta be minimized for preventing complications such as cerebral infarction, etc.

In view of the importance of the problems associated with conventional graft indwelling devices described in Patent Documents 1 and 2, the present inventors have made diligent studies for providing a stent that is remarkably improved in safety and curve-following capability during its delivery in the artery and in long-term stable indwelling in a diseased part, and as a result, they have proposed the following novel stent graft indwelling device (see Patent Document 3).

The stent graft indwelling device proposed above is a stent graft indwelling device 1 having a stent graft holding portion and having a dilator 10 having a fixed chip 20 and a sheath 30 loadable with a stent graft 60 held on the stent graft holding portion, and it is a stent graft indwelling device 1 having means of adjusting an insertion and/or indwelling position of the above stent graft 60 when the stent graft 60 is released from the above sheath 30 for indwelling. During the indwelling of the above stent graft 60 that is released from the above stent graft indwelling device and indwelled, the stent graft indwelling device can flex the stent graft 60 itself by utilizing the expansion power of the stent graft 60 and make the fixed chip 20 free from a blood vessel wall, thereby decreasing the impairment of the blood vessel and the freeing of an embolic substance from the blood vessel wall.

Further specific features of the (A) stent graft indwelling device described in Patent Document 3 are:
(1) When the stent graft 60 is released from the sheath 30, the wire 40 comes to be applying tension on the stent graft 60 to be released, thereby fine adjustment of the insertion angle and indwelling position can be made in a blood vessel while the stent graft 60 is in a semi-expansion or not completely expanded state.

(2) The stent graft 60 can be released into a blood vessel in a state where tension is applied so that the curvature of the stent is enhanced (toward a greater curvature) and it is decreased in length, so that even in the blood vessel with great curvature, smooth placement of the stent graft 60 can be made following the wall of the blood vessel.

(3) The leading end of the sheath 30 and the center side of the stent graft 60 can be freed from a blood vessel wall by utilizing or harnessing the expansion power of the stent graft 60 per se while the stent graft 60 is released, whereby the impairment of the blood vessel and the freeing or disengagement of an embolus substance from the blood vessel wall can be prevented during the maneuver of placing the stent graft.

(B) Further, since the fixed chip 20 to be mounted on the dilator 10 in the above stent graft indwelling device has a two-piece structure composed of the base body 21 and the cap 22, the hook holding groove can be constituted three-dimensionally, thus it has the following features.

(1) It can be manufactured on a large-scale with high form-reproducibility.
(2) (i) (A case where tension is exerted on the stent graft with the wire 40)
In the above fixed chip 20, when the stent graft 60 is housed in the sheath 30, the hook 66 on the leading end of the stent graft 60 in no case comes off from the fixed chip, and when the rear end portion of the stent graft 60 is released out of the sheath 30, the tension of the wire 40 is no longer at work, whereby the hook 66 can be detached easily.

### (ii) (A case where wire 40 is not used)

In the fixed chip of said invention, when the stent graft 60 is housed in the sheath 30, the hook 66 on the leading end of the stent graft 60 in no case comes off from the fixed chip, and when the rear end portion of the stent graft 60 is released out of the sheath 30, the hook 66 can be easily detached.

(3) In the fixed chip of said invention, the tubular (pipe-like) stent graft holder 14 passes through the two parts (base body 21 and cap 22) which are bonded and/or fitted to each other, whereby the cap 22 does not fall off the base body 21.

Unfortunately, however, in the stent graft indwelling device described in Patent Document 3, the insertion angle and placement site of the stent graft 60 are finely to be adjusted in a blood vessel in a semi-expansion state where the stent graft 30 is not completely expanded, so that it has a problem that operations therefor are not necessarily easy.

[Patent Document 1] JP 2000-262632 A
[Patent Document 2] JP 2000-350785 A
[Patent Document 3] International Publication WO2005/099806

### Disclosure of Invention

### Problems to be Solved by the Invention

The problem that this invention seeks to overcome is that with respect to the stent graft indwelling device described in Patent Document 3, since the insertion angle and placement position are finely adjusted in a blood vessel in a semi-expansion state where the stent graft 60 is not completely expanded, operations therefor are not necessarily easy.

### Means to Solve the Problems

For overcoming the above problem, therefore, the present inventor has made diligent studies and arrived at the following inventions.

[1] According to the invention, there is provided a stent graft indwelling device (1) having a dilator (10) having a stent graft holder (14) and a sheath (30) loadable with a stent graft (60) held on the stent graft holding portion (14) of said dilator (10),
   Said stent graft (6) being formed of a plurality of connected tubular units (61), the stent graft indwelling device having means of adjusting the insertion angle and/or indwelling position of said stent graft when said stent graft is released from said sheath (30) for indwelling,
   the means of adjusting the insertion angle and/or indwelling position of said stent graft (6) comprising a fixed chip (20) containing a contrast medium and/or a holding ring (69A) of a wire 40 attached to the rearmost one of said tubular unit (61) of said stent graft (60).

[2] According to the invention, there is provided a stent graft indwelling device (1) of [1] above, which has the dilator (10) having a fixed chip (20) in its forward end and a sheath (30) loadable with the stent graft (60) held on the stent graft holder (14) of said dilator (10),
   the stent graft indwelling device (1) being adapted to reduce impairment of a blood vessel and disengagement of an embolic substance from a blood vessel wall by flexing the stent graft (60) by harnessing the expansion power of the stent graft (60), thereby making the fixed chip (20) free from the blood vessel wall, while the stent graft (60) is released from the stent graft indwelling device (1) for indwelling.

[3] According to the invention, there is provided a stent graft indwelling device (1) of [1] or [2] above, wherein said dilator (10) has a base body portion (11), the stent graft holder (14) and the fixed chip (20),
   said dilator (10) has a through hole and a reinforcement tube (13) being attached to an inner circumference thereof,
   said reinforcement tube (13) is formed of a hard material (13A), a soft material (13B) and a hard material (13A) that are alternately arranged from a leading end portion to a proximal end portion.

[4] According to the invention, there is provided a stent graft indwelling device of any one of [1] to [3] above, wherein a wire lumen (100) for letting through a wire (40) to be used for adjusting the indwelling position and/or insertion angle of the stent graft (60) is formed in cross section of said dilator from the proximal end to the leading end of said dilator (10).

[5] According to the invention, there is provided a stent graft indwelling device of any one of [1] to [4] above, wherein the proximal end portion (12) of said dilator is provided with a fixing means for the wire (4) that is used for adjusting the indwelling position and/or insertion angle of the stent graft (60).

[6] According to the invention, there is provided a stent graft indwelling device (1) of any one of [1] to [5] above, wherein said sheath (3) has a forward end formed in the form of a curvature, and the forward end curvature form and a curvature radius R being selected from
   (A) [type A] and a curvature radius R of 140 to 170,
   (B) [type J] and a curvature radius R of 45 to 90,
   (C) [type LU] and a curvature radius R of 60 to 70, and
   (D) [type U] and a curvature radius R of 60 to 70.

[7] According to the invention, there is provided a stent graft indwelling device (1) of any one of [1] to [6], wherein said stent graft (60) is formed of a plurality of tubular units (61) connected in the center axis direction thereof, a holding ring (69) and a hook portion (66) are attached to an outer circumference of the first one of the tubular units (61), holding rings (67, 68) are attached to said hook portion (66) and a plurality of holding rings (69A) are attached to a rear portion of the rearmost one of the tubular units (61).

[8] According to the invention, there is provided a stent graft indwelling device of any one of [1] to [7], wherein said stent graft (60) is covered with a tubular member (65) and at least one opening portion (65A) is formed in said tubular member (65).

[9] According to the invention, there is provided a stent graft indwelling device of any one of [1] to [8], wherein said fixed chip (20) is composed of a base body (21) and a cap (22), a hook holding groove (26) is formed between said cap (22) and an upper opening portion of the base body (21), and the base body and the cap contain contrast medium in different amounts.

[10] According to the invention, there is provided a stent graft indwelling device (1) of any one of [1] to [9], wherein said wire (40) extends from an inside of the stent graft (6) to a wire fixing means formed in the proximal end portion (12) of the dilator through a lumen (100) formed from the base body portion (11) of the dilator (10) to the base portion (12) of the dilator and fixed, and, when the stent graft (60) is released from the sheath (30), said wire is capable of adjusting the release angle and indwelling position of the stent graft (60) by applying tension to compress the stent graft (60) with the wire (40) during the release of the stent graft (60).

[11] According to the invention, there is provided a stent graft indwelling device (1) of any one of [1] to [10], wherein a wire end portion (41) fixed to said fixing means is loosened and pulled out of the holding rings (67, 68, 69 and 69A) of said stent graft (60), whereby the wire can be pulled out of said stent graft indwelling device (1).

[12] According to the invention, there is provided a stent graft indwelling device (1) of any one of claims 1 to 11, wherein a forward end of the stent graft holder (14) is forced into a lumen (24) of proximal end portion (23) of the base body (21) and a lumen (25) of the cap (22), and the forward end portion of the above stent graft holder (14) passes through these lumens (24) and (25) to fix the cap (22) and the base body (21).

[13] According to the invention, there is provided a stent graft indwelling device (1) of any one of [1] to [12], wherein said means for adjusting the insertion angle and/or indwelling position of said stent graft (60) includes the wire fixing means attached to the base body portion (11) of the dilator (10), the stent graft holder (14) and the fixed chip (20) and the proximal end portion (12) of the dilator; a hook portion (66) formed in the leading end of the first tubular unit (61) of the stent graft (60), holding rings (67 and 68) formed in said hook portion (66), a plurality of holding rings (69A) formed in the front portion of the first tubular unit (61), a plurality of holding rings (69A) formed in the back portion of the tubular unit (61) forming the backmost row; and the wire (40) that passes through a wire lumen (100) formed between the holding rings (67,68,69,69A) and the dilator (10) and the sheath (30) or from the leading end (10) of the dilator (10) to the trailing end (12) of the dilator and a wire insertion inlet (82) and fixed with said wire fixing means.

[14] According to the invention, there is provided a stent graft (60) loadable in the dilator (10) of said stent graft indwelling device (1) recited in any one of [1] to [13], which is constituted of a plurality of tubular units (61) connected in the central axis direction thereof, a holding ring (69) and a hook portion (66) being attached to an outer circumference of a first one of the tubular units (61), and holding rings (67 and 68) being attached to said hook portion (66),
   a plurality of holding rings (69A) being attached to a back of the rearmost one of said plurality of the tubular units (61).

[15] According to the invention, there is provided a fixed chip (20) that is to be attached to the dilator (10) of the stent graft indwelling device (1) recited in any one of [1] to [13] and formed of a base body (21) and a cap (22), a hook holding groove (26) being formed between the cap (22) and an upper opening portion of the base body (21), the base body (21) and the cap (22) containing a contrast medium in different amounts.

[16] According to the invention, there is provided a stent graft indwelling device (1') having a dilator (10) having the fixed chip (20') and a sheath (30) loadable with a stent graft (60) held on a stent graft holder (14) of said dilator (10), wherein in a state where the rearmost tubular member (61) of the stent graft (60) is folded into the sheath (30), the largest diameter S1 of backward end of the housed tubular member (61) is smaller than the largest diameter S2 of forward end of the base body portion (11) of the dilator (10) loaded in the sheath (30).

### Advantageous Effects of the Invention

This invention has the following advantageous effects.
(1) In this invention, a plurality of holding rings 69A of a wire are attached to the backward end of the rearmost tubular unit 61 of the stent graft 60 as means of adjusting the insertion angle and/or indwelling position of the stent graft 60, so that the release and indwelling of the stent graft in the blood vessel are made easy when the above stent graft 60 is to be indwelled in a blood vessel.

(2) The fixed chip 20 having the base body 21 and the cap 22 containing different amounts of a contrast medium is employed as means of adjusting the insertion angle and/or indwelling position of the stent graft, so that the base body 21 and the cap 22 are clearly distinguishable in contrast during imaging procedure, and the position of the hook 66 (of the stent graft 60) engaging with the hook holding groove 26 can be clearly monitored. Therefore, the maneuver of fixing the stent graft (adjustment of the insertion angle and the indwelling position) is made easy.

(3) The reinforcement tube 13 is provided on the inner circumference of the dilator 10, and the hard material 13A, the soft material 13B and the hard material 13A are alternately arranged from the leading end portion to the proximal end portion of the above reinforcement tube, so the property of highly tracking a blood vessel can be improved even when the blood vessel is curved and irregular.

(4) Since the wire lumen 100 formed in a cross section of the base body 11 of the dilator 10 from the proximal end to the leading end, the removal of the wire can be easily removed.

(5) The forward end of the sheath 30 is formed in a curved form, the above forward end form the curvature radius R thereof are formed to satisfy one of the followings: (A) "type A", a curvature radius R: 140 - 170, (B) "type J", a curvature radius R: 45 - 90, (C) "type LU", a curvature radius R: 60 - 70, and (D) "type U", a curvature radius R: 60 - 70. Therefore, the sheath can be easily inserted even into an irregular blood vessel, and the curve-following capability of the blood vessel can be improved.

(6) Since at least one opening portion is formed in the tubular member 65 covering the stent graft 60, it can be ensured that blood easily flows in small blood vessels (brachiocephalic arterial trunk, left common carotid artery and left subclavian artery) branched from aorta thoracica.

(7) In this invention, the stent graft 60 is formed by covering the stent, and in a state where the tubular member 61 constituting the proximal or rear end of the stent graft 60 is folded and housed in the sheath 30, it is adjusted to ensure that the largest diameter S1 of the backward end of the rearmost tubular member 61 is smaller than the largest diameter S2 of the leading end of the dilator loaded in the sheath, whereby the adjustment of the stent graft to the fixing position in celiac artery branched from aorta thoracica can be accurately performed.

### Brief Description of Drawings

Fig. 1 is a schematic drawing of the stent graft indwelling device of this invention.
Fig. 2 is an enlarged cross-sectional view taken along line A-A in Fig. 1.
Fig. 3 is an enlarged cross-sectional view taken along line C-C in Fig. 1.
Fig. 4 is an exploded view of the stent graft indwelling device of this invention (showing that the device comprises a dilator 10, a sheath 30, a stent graft 60 and a wire 40).
Fig. 5 shows cross-sectional views of the dilator 10 for use in this invention. In Figure, (A) shows a longitudinal cross-sectional view in the vicinity of A-A in Fig. 1, and (B) shows a longitudinal cross-sectional view in the vicinity of C-C in Fig. 1.
Fig. 6 shows schematic drawings showing Examples [(A), (B), (C) and (D)] of the form of leading end of the sheath 30 for use in this invention.
Fig. 7 is a schematic drawing of a stent graft for use in this invention.
Fig. 8 shows schematic drawings of a stent graft for use in this invention. In Figure, (A) is a schematic drawing of the stent graft (two wire holding rings 69 being formed at a back of the stent graft 60), in which the wire is set through, and (B) is a partially enlarged view of (A).
Fig. 9 shows schematic drawings of the stent graft for use in this invention, in which (A), (B) and (C) show the opening portion(s) 65A formed through a tubular member 65.
Fig. 10 shows schematic drawings for explaining the fixed chip 20 of this invention, in which (A) is a plan view, (B) is a front view, (C) is a bottom view, (D) is a left side view, (E) is a right side view, and (F) is a partially enlarged cross-sectional view.
Fig. 11 shows schematic drawings for explaining the fixed chip 20 of this invention, in which (A) is a plan view, (B) is a front view, (C) is a bottom view, (D) is a left side view, and (E) is a right side view.
Fig. 12 shows schematic drawings for explaining the fixed chip 20 of this invention, in which (A) is a plan view, (B) is a front view, (C) is a bottom view, (D) is a left side view, and (E) is a right side view.
Fig. 13 shows schematic drawings for explaining a fixed chip 20' of this invention, in which (A) is a plan view, (B) is a longitudinal cross-sectional view, and (C) shows a state where a hook 66 of the stent graft 60 is mounted.
Fig. 14 is a drawing for explaining one example of a method of operating the stent graft indwelling device of this invention.
Fig. 15 is a drawing for explaining one example of a method of operating the stent graft indwelling device of this invention.
Fig. 16 is a drawing for explaining one example of a method of operating the stent graft indwelling device of this invention.
Fig. 17 is a drawing for explaining one example of a method of operating the stent graft indwelling device of this invention.
Fig. 18 is a drawing for explaining one example of a method of operating the stent graft indwelling device of this invention.
Fig. 19 is a drawing for explaining one example of a method of operating the stent graft indwelling device of this invention, in which (A) is a schematic drawing for explaining the operating method, and (C) and (D) are partially enlarged views of (A).
Fig. 20 is a drawing for explaining one example of a method of operating the stent graft indwelling device of this invention.
Fig. 21 is a drawing for explaining one example of a method of operating the stent graft indwelling device of this invention.
Fig. 22 is a drawing for explaining one example of a method of operating the stent graft indwelling device of this invention.

### (Explanation of Numerals)

- 1, 1': Stent graft indwelling device
- 10: Dilator
- 11: Base body portion of dilator
- 12: Proximal end portion of dilator
- 13: Reinforcement tube
- 13A: Hard material
- 13B: Soft material
- 14: Stent graft holder
- 17: Fluid passage (concave portion)
- 20, 20': Fixed chip
- 21, 21': Fixed chip base body
- 22: Cap for fixed chip
- 22A: Cap body
- 23, 23': Base body proximal end portion of fixed chip
- 24, 24': Lumen of base body proximal end portion
- 25: Lumen of cap
- 26: Hook holding groove
- 26': Hook attaching groove
- 27: Contrast medium ejecting port
- 28: Top portion of cap
- 29: Engagement portion of cap
- 30: Sheath
- 31: Hub
- 32: Liquid inlet
- 37: Hub cap with valve
- 39: Enlarged diameter portion
- 40: Wire
- 41: End portion of wire
- 42: End portion of wire
- 60: Stent graft
- 61: Tubular units
- 61(1): Tubular unit
- 61(2): Tubular unit
- 61(3): Tubular unit
- 61(4): Tubular unit
- 61(5): Tubular unit
- 62: Flexed portion
- 63: Connecting portion
- 64: Stent
- 65: Tubular member (graft)
- 65A: Opening portion
- 66: Hook portion
- 67: Holding ring of hook portion
- 68: Holding ring of hook portion
- 69: Holding ring
- 69A: holding ring
- 80: Branch tube
- 81: Insertion inlet for guide wire
- 82: Insertion inlet for wire
- 83: Fixed cap
- 101: Wire lumen
- 101: Guide wire lumen

### Best Mode for Carrying Out the Invention

This invention will be explained in detail below with reference to drawings.

### (Stent graft indwelling device)

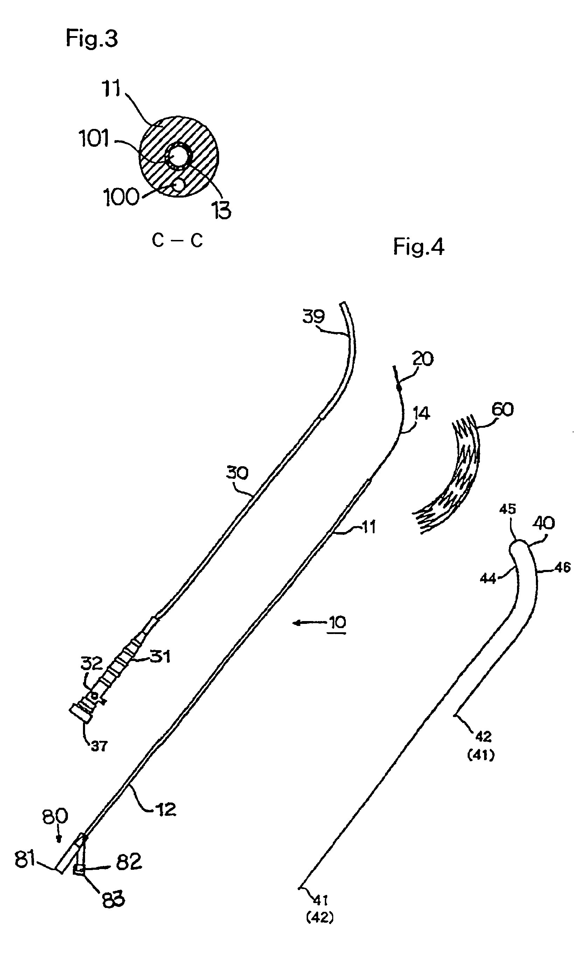

The stent graft indwelling device 1 of this invention is an indwelling device, for example, as shown in the exploded view of Fig. 4, and it comprises a dilator (pushing rod) 10 having a stent graft holder 14 and a sheath 30 to be loaded with a stent graft 60 held on the stent graft holder 14 of the above dilator 10. The basic feature is that the device has means for adjusting the insertion angle and/or indwelling position of the above stent graft 60 when the above stent graft 60 is released from the above sheath 30 and placed at the position or site.

In this invention, the means for adjusting the insertion angle and/or indwelling position or site of the above stent graft 60 is realized or achieved by a wire 40 and/or a fixed chip 20 mounted on the dilator 10.
The most characteristic feature of this invention is that the means for adjusting the insertion angle and/or indwelling position of the above stent graft 60 is realized or achieved by a holding ring 69A mounted (formed) in a tubular unit 61 forming the backmost row of the stent graft 60 and/or the fixed chip 20 to which a contrast medium or imaging agent is added.

As shown in Fig. 4, the feature of the indwelling device of this invention is that it is the stent graft indwelling device 1 having the dilator 10 having the fixed chip 20 and the sheath 30 loaded with the stent graft 60 held on the stent graft holder 14 of the above dilator 10 and that while the stent graft 60 is released from the stent graft indwelling device 1 for indwelling, the stent graft 60 itself becomes flexed through the expanding power of the stent graft 60 thereby making the above chip 20 free from a blood vessel wall, and whereby reducing the damage to the blood vessel and the disengagement of an embolic or a thrombolic substance from the blood vessel, as will be described later.

The "means for adjusting the insertion angle and/or indwelling position of the stent graft 60" includes the wire 40 and/or the fixed chip 20 in a broad sense. More specifically, the "means for adjusting the insertion angle and/or indwelling position of the stent graft 60" with respect to (i) the fixed chip 20 first includes a base body portion 11 of the dilator 10, the stent graft holder 14 and the fixed chip 20 (to which a contrast medium is added) mounted on the leading end of the above holder 14 shown in Fig. 4.

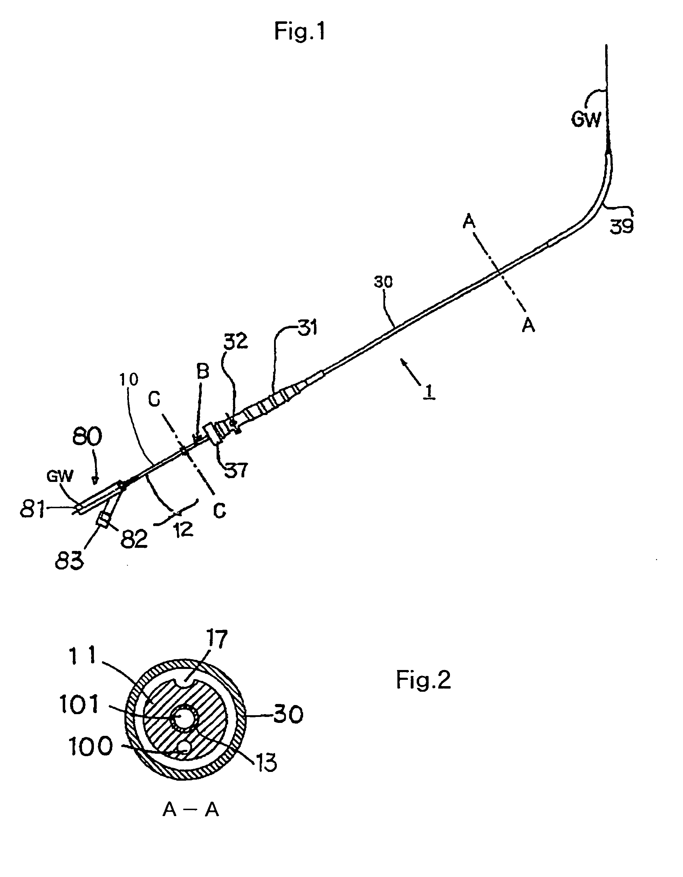

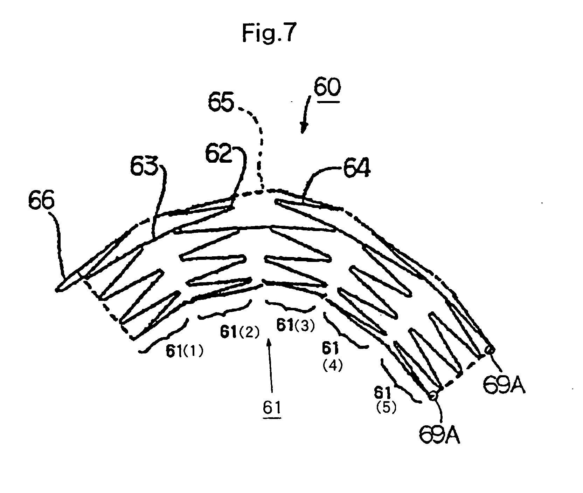

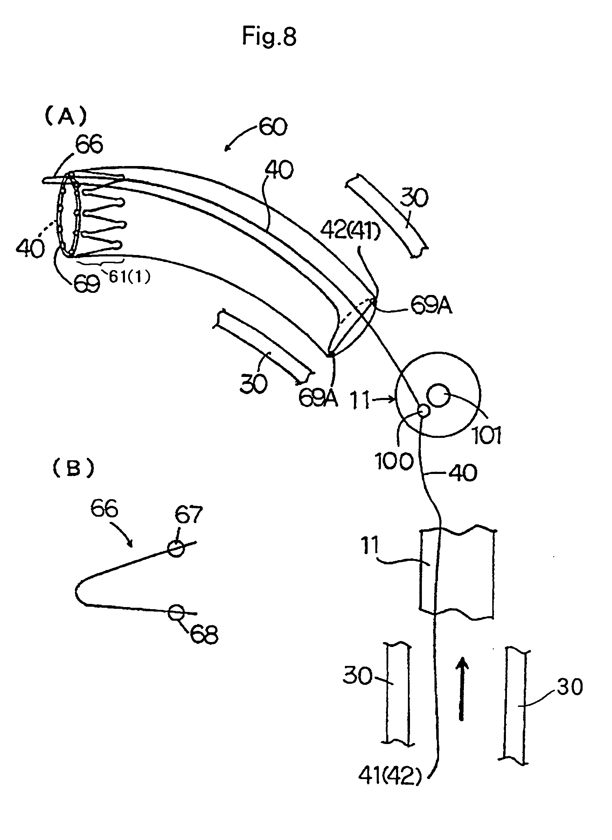

Further, with respect to (ii) the wire 40 (or wire system), it is included a hook portion 66 in the leading end of the first tubular unit 61 of the stent graft 60, holding rings 67 and 68 formed in the above hook portion 66, a plurality of holding rings 69 formed in the front portion of the first tubular unit 61, a plurality of holding rings 69A formed in the back portion of rearmost one of the tubular units 61, and wherein the wire 40 being inserted between the dilator 10 and the sheath 30 from the above holding rings 67, 68, 69 and 69A or into a wire lumen 100 of the dilator 10 shown in Figs. 7 and 8. The wire lumen 100 is formed in the cross section of the dilator 10 all the way from the leading end of the dilator 10 to the proximal end portion 12 of the dilator (see Figs. 2 and 3).

### (Dilator 10)

The dilator 10 is shown in Fig. 4 as described above and has the base body portion 11, the stent graft holder 14 and the fixed chip 20. The stent graft holder 14 has a smaller diameter than the base body portion 11 for mounting the stent graft 60 on the outer circumference thereof.

Further, the dilator 10 has a guide wire lumen 101 for passing a guide wire GW in the vicinity of its center as shown in Fig. 2 (cross-sectional view taken along A-A in Fig. 1) or Fig. 3 (cross sectional view taken along C-C in Fig. 1) as will be described later, while a reinforcement tube 13 (to be also referred to as "inner tube") is fitted to the inner circumference of the above lumen portion.

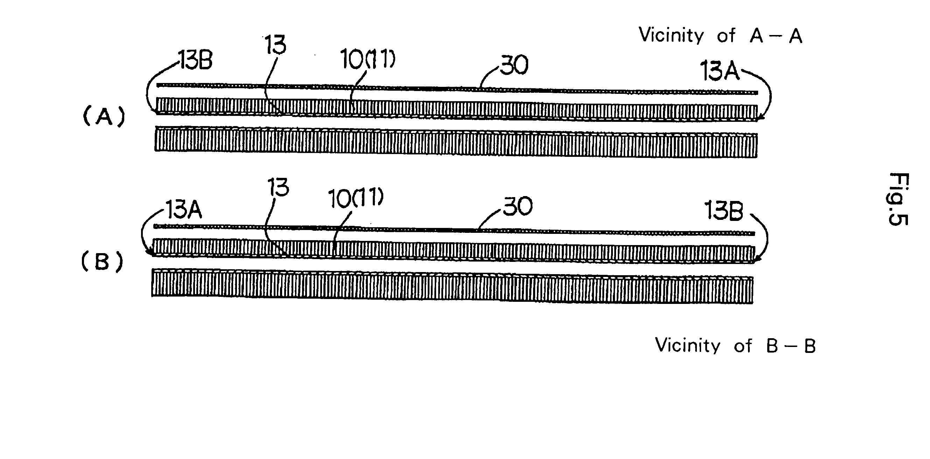

The above reinforcement tube 13 has an arrangement of a hard material 13A, a soft material 13B and a hard material 13A from the leading end portion to the proximal end portion as shown in Fig. 5 [(A) is a longitudinal cross-sectional view in the vicinity of the cross-sectional view taken along A-A in Fig. 1 and (B) is a longitudinal cross-sectional view in the vicinity of the cross-sectional view taken along C-C in Fig. 1]. For example, metals [such as a pipe formed of stainless steel (SUS), etc.] are preferred as the hard material 13A, and soft plastics (such as pipes formed of polypropylene, polyethylene, etc.) are preferred as the soft material 13B. The constitution wherein soft material is arranged between the hard materials as described above can improve the curvature-following capability of the tube to a curved blood vessel.

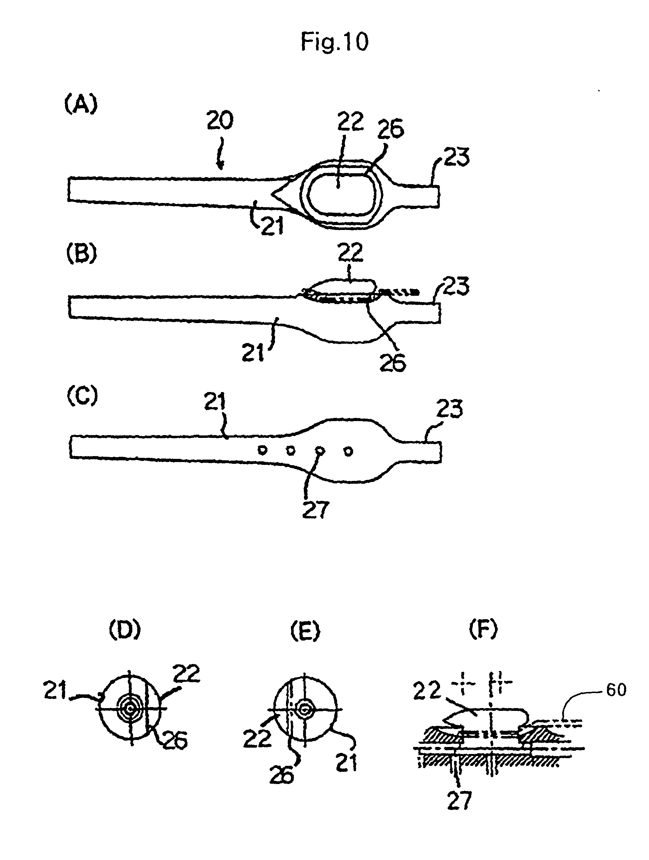

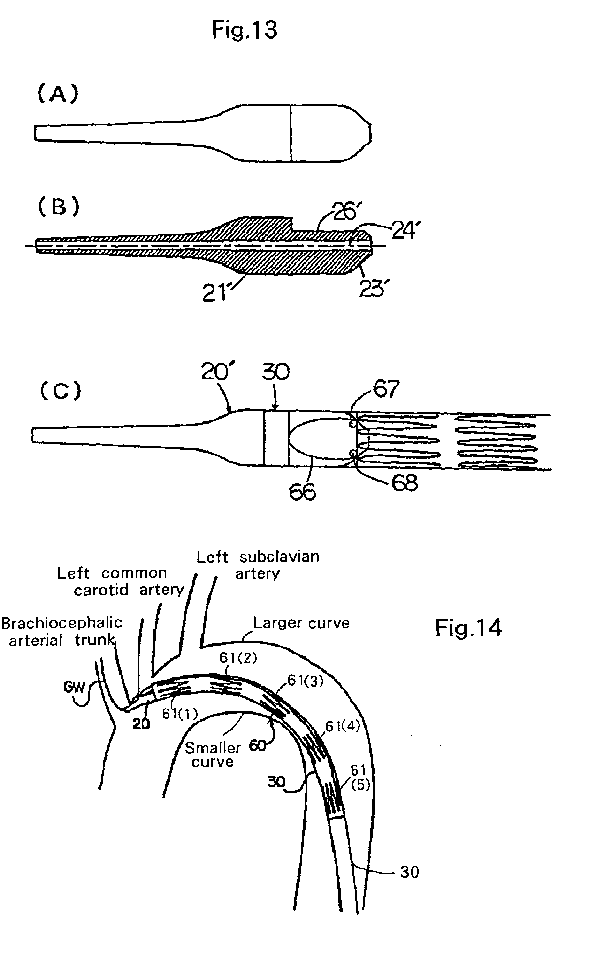

The above base body portion 11 and the stent holder 14 are formed, for example, from a SUS pipe (rod or bar) or a rigid material such as a hard resin.
Further, the fixed chip 20 (Figs. 10 to 12) or 20' (Fig. 13) is mounted on or attached to the leading end or distal end portion of the above stent graft holder 14. The above chip 20 or 20' has a form as shown in Figs. 10 to 13 and is formed from a material having a proper hardness and flexibility among synthetic resins including polyamide elastomer, polyurethane, polyvinyl chloride, etc., as will be described in detail later.

The dilator 10 has in its cross section the wire lumen 100 for passing the wire 40 and the guide wire lumen for passing the guide wire GW, the wire lumen and the guide wire lumen being formed from the proximal end portion to the leading or distal end portion as shown in Figs. 2 and 3.

As shown in Figs. 2 and 3, further, the dilator 10 has a nearly Y-letter-shaped branch tube 80 formed in its proximal end portion, and in the branch tube 80, there are formed an insertion inlet 81 for the guide wire GW and an insertion inlet 82 for the wire 40. Fitted to the insertion inlet 82 for the wire 40 is a fixing cap 83 for the wire 40.

The base body portion 11 of the dilator 10 is in principle formed of a solid rod, while it is not completely solid, and a fluid passage 17 in a concave form is formed on a side of the base body potion 11 as shown in Fig. 2. The thus formed fluid passage 17 enables saline solution to flow in the above fluid passage 17 before the indwelling device is intracorporeally inserted, thereby evacuating and remove air from the sheath 30. Further, the wire 40 can be passed through the above fluid passage 17.

### (Fixing means for wire 40)

In this invention, the wire 40 is used for adjusting the indwelling position or site and the insertion angle of the stent graft 60 as will be described later.
The fixing means for the wire 40 refers to a means that is capable of fixing one end portion 41(42) of the wire 40 by nipping or pinching, and capable of letting loose the nipping or pinching pressure when the fixing of the wire end 41 (42) is to be released.

For example, a specific example of the wire fixing means refers to the branch tube 80 shown in Figs. 1 and 4. The branch tube 80 has the insertion inlet 82 for the wire 40 and the fixing cap 83, and one end 42(41) of the wire 40 can be fixed between the insertion inlet 82 and the fixing cap 83 by means of the insertion inlet 82 and the fixing cap 83 of the branch tube 80.

The fixing means for the wire 40 is also formed of the stent graft 60 and the sheath 30 as shown in Fig. 8(A). That is, one end 42(41) can be fixed by pinching it between an outer circumference of the stent graft 60 and an inner circumference of the sheath 30.

### (Sheath)

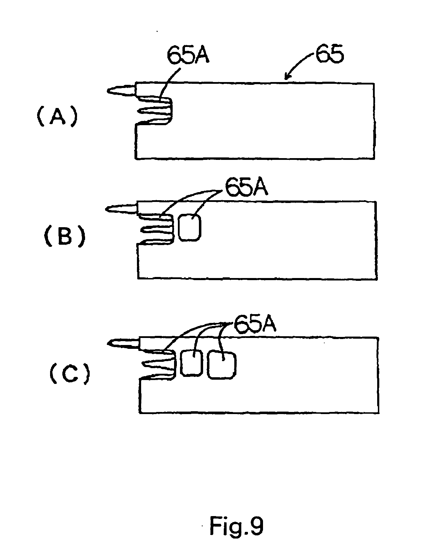

The sheath 30 is formed of a tube having such an inner diameter that it can house or accommodate the above dilator 10 and the stent graft 60 (more specifically, the dilator 10 holding the stent graft mounted on the stent graft holder 14) as shown in Fig. 9.

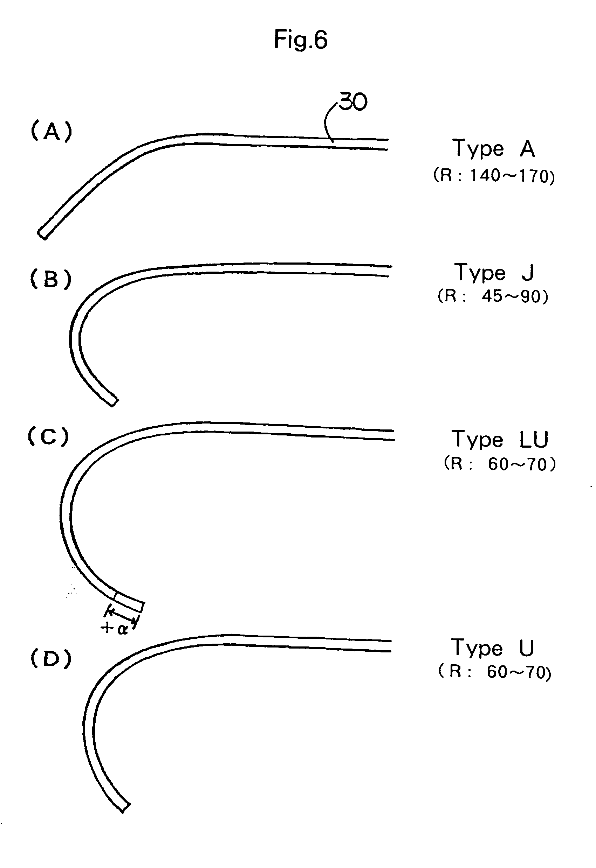

Further, the leading or distal end portion of the sheath 30 is curvedly formed so that it can be easily inserted into a curved blood vessel of a patient. For example, it is curvedly formed in the form of (A), (B), (C) or (D) shown in Fig. 6. The degree of the curvature is adjusted and defined depending upon the form (type) of the curvature and a curvature radius R.
For example, Fig. 6(A) shows what is called "type A", and the curved portion is formed so as to have a curvature radius of 140 to 170. Fig. 6(B) shows what is called "type J", and the curved portion is formed so as to have a curvature radius of 45 to 90. Fig. 6(C) shows what is called "type LU", and the curved portion is formed so as to have a curvature radius of 60 to 70. Fig. 6(D) shows what is called "type U", and the curved portion is formed so as to have a curvature radius of 60 to 70. The "type LU" and the type "U" have similar forms (the same curvature radius R), while the difference between the type "LU" and the type "U" is that the type "LU" is formed so as to have a longer leading end portion by +α (e.g., 20 mm).

Further, a hub 31 is mounted on the proximal end portion of the sheath 30, and the hub 31 is provided with a liquid inlet 32. On the other hand, an enlarged diameter (or expanded diameter) portion 39 is formed in the leading or distal end portion of the sheath 30, and the stent graft 60 held on the stent graft holder 14 is accommodated or arranged inside the enlarged diameter portion 39. The sheath 30 is formed from a flexible material. The flexible material can be selected, for example, from simple synthetic resins such as a fluorocarbon resin, a polyamide, a polyamide elastomer, polyvinyl chloride, etc., compounds of these resins, multi-layered structures of these resins, or further, composite materials of these resins with metal wire or the like.

Further, as shown in Figs. 1 and 4, a hub cap 37 with a valve is mounted on the base portion of the hub 31 in order to ensure that no blood leaks when the dilator 10 is inserted into the sheath 30.

### (Stent and stent graft)

The stent graft 60 in this invention is obtained by covering a stent 64 with a tubular member 65 formed of a flexible fiber or film of a synthetic resin or the like as shown in Fig. 7. Like a stent, the stent graft is used for repairing a blood vessel that suffers damage such as stenosis or aneurysm or as a substitute for a hollow organ such as an artificial vessel or the like.

As the stent 64 that constitutes the structure or framework of the stent graft 60 for use in this invention, for example, a stent shown in Fig. 7 is used. The stent 64 as is shown in Fig. 7 is obtained by bending a metal wire in a zigzag form to prepare a tubular (annular) unit 61 formed of zigzag patterns, arranging a plurality of such tubular units 61 in the form of a tubular circumference so as to surround the central axis of the stent, connecting each of the tubular units at least in one place each by known means such as welding, soldering or calking to prepare tubular units, and connecting a plurality of such annular units through connecting portions (connecting lines) (connecting struts) 63 in the central axis direction of the annular product. In Fig. 7, for example, five tubular units 61 (61(1), 61(2), 61(3), 61(4) and 61(5) are connected. The number of the tubular units is not limited to five and may be any number.

The stent graft is obtained by covering the outer circumference of the stent 64 with the above flexible tubular member 65 (e.g., Dacron fiber (polyethylene terephthalate fiber, trade name of Du Pont de Nemours, Co., Ltd.) or a film formed of a fluorocarbon resin such as PTFE (polytetrafluoroethylene) as described above. The above tubular member 65 is sutured and fixed to flexed portions 62 (that is the flexed or bending portions of the zig-zag patterns constituting the stent) of leading and proximal ends of the stent 64, for example, with a wire such as a blood vessel suture.

The base material for the metal wire that forms the stent 64 is not specially limited, and it can be selected from generally used materials including stainless steel such as SUS316L, etc., superelastic alloys such as Ti-Ni alloy, etc., titanium, titanium alloy, tantalum, tantalum alloy, platinum, platinum alloy, tungsten, tungsten alloy and the like.

As the above stent graft 60, there can be preferably used a stent graft having the form or configuration proposed by the present inventors in JP 2003-334255 A.
That is, the technical features of the above publication are incorporated by reference into the disclosure of the present specification. However, the stent graft 60 shall not be limited thereto, and there may be used any stent graft that is known, and any stent graft can be used as long as it at least has the form shown in Fig. 7 or any other form capable of constituting a stent framework structure such as a mesh form, a spiral form, etc.

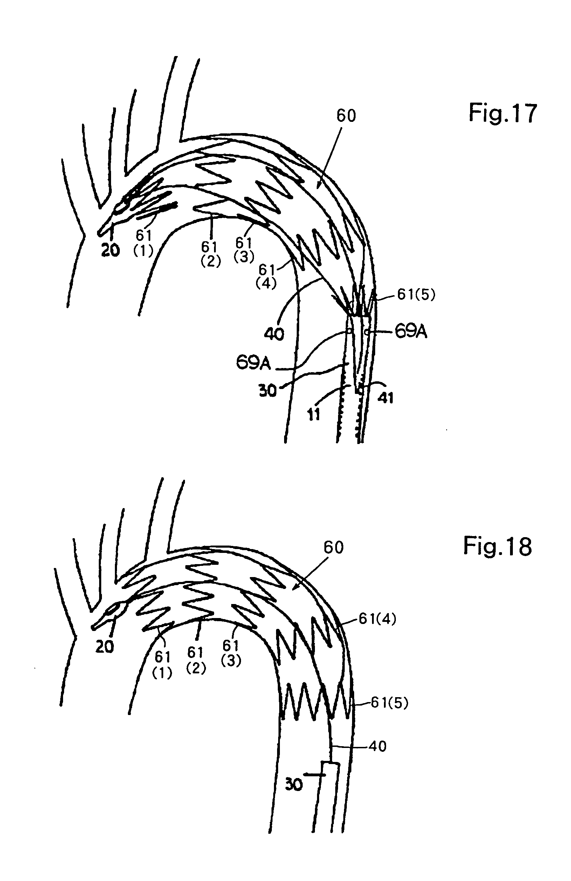

The stent graft 60 that is preferably used in this invention is as follows. In an ordinary state, where it is not inserted into the sheath 30, it has a curved form that is curved along the longitudinal direction of the stent graft 60 as shown in Fig. 7 (a curved configuration in an ordinary state). When it is inserted or accommodated into the sheath 30, it is contracted or compressed in the central axis direction of the stent 64 forming a framework structure, and in the sheath 30, it is housed or accommodated in the form of a curvature along the enlarged diameter portion 39 or the stent graft holder 14 of the sheath. And, the stent graft housed in the sheath 30 in this state is delivered or transferred to a diseased part of the blood vessel together with the sheath 30 and there it is released from the sheath. After released, the stent expands outwardly in the radius direction to become the stent 64 in an indwelling state, holding the curved and expanded form along the longitudinal direction of the stent 64 in a curved ordinary state, as will be discussed with reference to Fig. 18.

The above curved configured stent graft 64 (i.e., stent 60 as a framework structure) as shown in Fig. 7 can be arranged in a three-dimensionally curved state along the length direction by shifting the position of connecting portion 63 between tubular units 61 following the procedure as disclosed in JP 2003-334255A. Specifically, one tubular unit is connected to another tubular unit with two or more connecting portions (connecting struts), and the space (distance) between the connecting portions is so changed, thereby the degree of curvature can be adjusted. With an increase in the space (distance) between the connecting portions, the curvature degree increases.

The stent graft 60 has, as its framework structure, the stent 64 thus having itself a spring action and is highly flexible, and the framework structure is covered with the tubular members 65 formed of a fibrous or film-like synthetic resin, so that the stent graft can freely follow the three-dimensional curvature thus of a blood vessel as required.

As shown in Fig. 8(A), further, a plurality of holding rings 69 and one hook portion 66 are attached to the outer circumference of the first tubular unit 61 (61(1)) forming the leading end (viewed in the insertion direction) of the stent graft 60 (stent 64). Further, two holding rings 67 and 68 are attached to the hook portion 66 as shown in Fig. 8(B).
Further, a plurality of (or two) holding rings 69A are attached to the back of the tubular unit 61 forming the proximal or rear end.

The holding rings 67, 68, 69 and 69A are formed in the form of a small circle each through which the wire 40 can pass. The above holding rings 69 and 69A may be formed in the form of a small circle each by rounding each flexed portion 62 (turning-up or bending portion of the zigzag pattern) forming the forward end of the stent 64, or by rounding a wire (not shown) such as a suture in the form of a small circle.

Each of the holding rings 67 and 68 to be attached to the hook portion 66 is made by rounding a metal wire or the like in the form of a small circle.
The hook portion 66 is formed in the form of a wing (also referred to as a fin-like form) as shown in Fig. 8 and can be made, for example, of a metal wire.
The holding rings 67, 68, 69 and 69A and the hook portion 66 can be made from a material (wire material) that is the same as, or different from, that which is used for the stent 64.

In the stent graft 60 shown in Fig. 7 and Fig. 8(A), generally, the entire surface of the stent 64 is covered with the tubular members 65. However, when the outer circumference of the stent graft 60 comes in contact with the position of a branch blood vessel as shown in Fig. 18 as will be discussed later, preferably, the tubular member 65 of the stent graft 60 is partly cut in the form of a U or V letter to form an opening portion (an outlet of blood flow) so that the stent framework structure is exposed to ensure that the branch blood vessel is not blocked.

In Figs. 9, (A), (B) and (C) show preferred embodiments of the tubular member 65 having such opening portion(s) 65A formed, and one to three opening portions 65A are formed in the tubular member 65. The formation of the above opening portion(s) 65A allows blood to flow in branched small blood vessels (brachiocephalic arterial trunk, left common carotid artery and left subclavian artery) shown in Fig. 14 through the opening portion(s) 65A. The form and number of the opening portions 65A can be adjusted depending upon the shape and state of small blood vessels of each patient.

### (Wire 40)

This invention has a characteristic feature in that the wire 40 is used for adjusting the indwelling position and the insertion angle of the stent graft 60, and the adjustment of the dwelling position and insertion angle of the stent graft 60 are made by adjusting the tension on the wire 40.

In principle, the wire 40 is constituted as shown in Fig. 4. That is, the wire 40 has a constitution in which its portion starting from its end portion 41(42) and corresponding to the linear base body portion 11 of the dilator is extended to form a straight-line portion that is in parallel with, or along, the base body potion 11, its subsequent portion corresponding to the curved stent graft holder 14 (or the curved stent graft 60) forms a curved portion 44 forming a curved structure matching the curve, its further subsequent portion that is connected to the hook 66 and the holding rings 67, 68, 69 and 69A forms a flexed portion 45, its portion following the above flexed portion turns back and similarly forms a curved portion 46 and its portion thereafter again forms a straight-line portion corresponding to the base portion 11 of the dilator and ends at the other end portion 42 (41).

One end 41(42) above is pulled out of the insertion 82 through a wire lumen 100 that passes through the dilator base body portion(or base portion) shown in Figs. 2 and 3, and fixed with the insertion inlet 82 and the fixing cap 83. The other end 42(41) is pulled out of the rear end of the stent graft 60 and fixed between the stent graft 60 and the sheath 30 by attaching the sheath 30 to the outer circumference of the stent graft 60.

More specifically, the wire 40 (flexed portion 45 thereof) is wound around the forward end circumference of the first tubular unit 61 (viewed from the insertion direction toward a patient) of the stent graft 60 along the above holding ring 69 as shown in (A) of Fig. 8(A). (Note: The wire is not completely bound or fixed to the holding rings 67, 68, 69 and 69A, thereby allowing the wire to move or slide along the rings).

In the above manner, the wire 40 is made to pass through the above holding rings 67, 68, 69 and 69A, and wound around the forward end circumference of the first tubular unit 61 (viewed from the insertion direction toward a patient) of the stent graft 60 nearly in the form of a ring, and two parts of the wire 40 (part of the wire toward the front portion and the other part of the wire from the front portion) are passed through the inside of the stent graft 60 symmetrically in the longitudinal direction. Then, one end 42(41) of the wire 40 is fixed between the outer circumference of the stent graft 60 and the sheath 30, and the other end 41(42) is pulled out of the insertion inlet 82 through the wire lumen 100 and fixed between the insertion inlet for the wire 40 and the fixing cap 83.

By any one of the above fixing methods, the wire 40 is so structured as to be easily taken out when the wire end portion 41 is pulled out of the wire insertion inlet 82.
In this invention, the material for constituting the wire 40 is not specially limited so long as it is a wire having strength and elasticity to some extent. It can be selected from sutures formed of a nylon fiber, a fluorocarbon resin fiber, etc., metals such as a nickel-titanium superelastic alloy, stainless steel, etc., or wires made of plastics, carbon, etc. For example, a nickel-titanium superelastic metal wire having a diameter of approximately 0.1 to 0.3 mm, a twisted stainless steel wire having a diameter of approximately 0.2 to 0.3 mm or a suture having a diameter of approximately 0.1 to 0.2 is suitably used.

### (Catheter)

The stent graft indwelling device of this invention can be used with a catheter mounted thereon. The catheter refers to a tube formed of a polyamide, polyurethane, etc., and has a guide wire lumen and a contrast medium or imaging agent lumen, and is connected to the top of the fixed chip 20 when used.
The stent graft indwelling device is generally inserted along a wire for guiding (guide wire GW) that is pre-inserted into a body along a route (from a brachial artery to a femoral artery through a branchiocephalic arterial trunk, an aortic arc, a thoracic aorta, an abdominal aorta, a common iliac artery and an external iliac artery). If the catheter is connected to the top end of the fixed chip, there is provided an advantage of decreasing the contact of the above top end to a blood vessel wall when the stent graft is passed through a curved artery. However, the stent graft indwelling device of this invention can be in many cases operated at a diseased part while keeping or maintaining the top end of the fixed chip from coming in contact with a blood vessel wall even if the above catheter is not mounted, thereby making it unnecessarily to mount the catheter thereon.

### (Fixed chip)

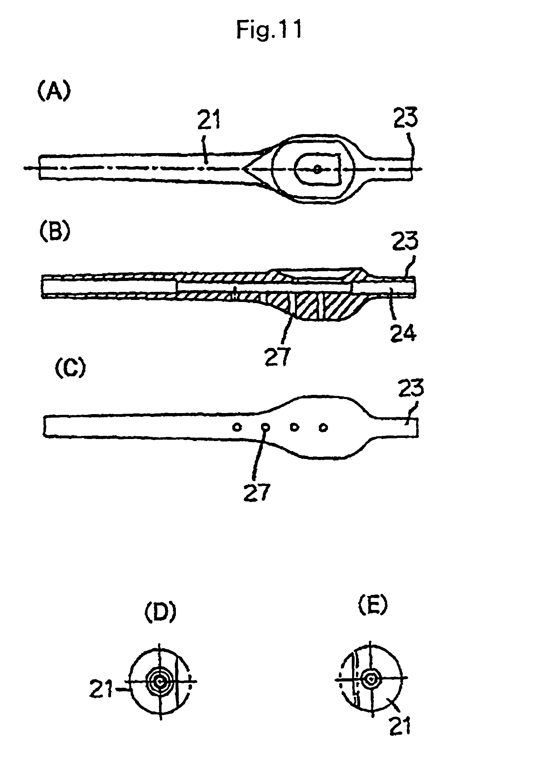

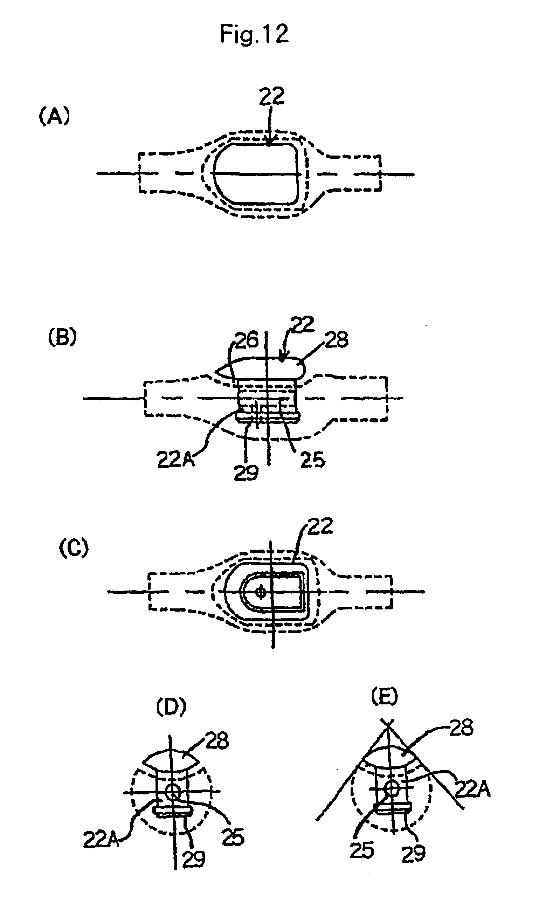

This invention also has as another technical feature in that the fixed chip 20 is attached to the forward end of the stent graft holder 14 to adjust the indwelling position and insertion angle of the stent graft 60.
The fixed chip 20 is in principle composed of two pieces such as a base body 21 and a cap 22. More specifically, as shown in Fig. 10 illustrating the base body 21 and the cap 22, Fig. 11 illustrating the base body 21 and Fig. 12 illustrating the cap 22, a hook holding groove 26 is formed between the upper opening portion of the base body 21 and the cap 22. Further, a contrast medium (imaging agent) ejecting port 27 is formed in the bottom of the base body 21.
In each of Figs. 12 to 14, (A) is a plan view, (B) is a front view (or longitudinal cross-sectional view), (C) is a bottom view, (D) is a left side view, (E) is a right side view, and (F) is a partial enlarged cross-sectional view.

As shown in Fig. 12, the cap 22 is composed of a body portion 22A and a top portion 28 covering the upper portion of the body portion, and in the bottom of the above body portion 22A is formed an engagement portion 29 to engage with the base body 21. In the body portion 22A, further, a lumen 25 to work as a flow passage for a contrast medium, etc., is formed. The cap 22 is attached to the base body 21 by causing the above engagement portion 29 (Fig. 12 shows a projection, while it may be a groove) to engage with an engagement portion (which is not shown, while it may be a groove or projection so long as it can engage with the engagement portion 29) of the base body 22.

In the fixed chip 20 for use in this invention, a contrast medium (e.g., barium sulfate) is added (incorporated) to the base body 21 and the cap 22, thereby the position of the fixed chip 20 can be monitored through contrast imaging procedure. In this case, the amounts of the contrast medium to be added the base body 21 and the cap 22 are adjusted (for example, 40 mass% to the base body 21, and 10 mass% to the cap 22), and the base body 21 and the cap 22 are caused to form images different in image density during contrast imaging procedure, thereby distinguishing of the base body and the cap is made.

More specifically, the difference between the base body 21 and the cap 22 in contrast is made clear during contrast imaging procedure, the position of the hook 66 (of the stent graft 60) engaged with the hook holding mechanism 26 is easily confirmed. The maneuver of fixing the stent graft 60 (adjustment of the insertion angle and indwelling position) can be made easier.

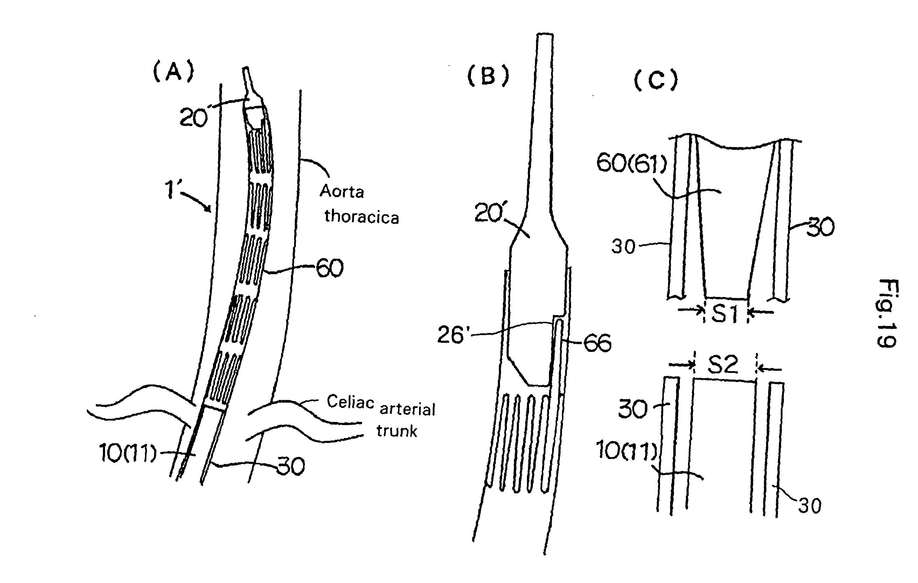

In this invention, a fixed chip without a cap can be also used. Fig. 13 shows an embodiment of a fixed chip 20' without a cap, and the fixed chip 20' is used for positioning the rear end of the stent graft 60 to a celiac arterial trunk as will be described later with reference to Figs. 19 to 22.

The fixed chip 20'shown in Fig. 13 has substantially the same form and structure as those of the fixed chip 20 shown in Figs. 11 and 12 except that it is formed of one piece (integrally formed) and is without the cap 22 and the contrast medium ejecting port 27 and that a groove 26' for attaching the hook 66 (of the stent graft 60) thereto is formed in the rear top portion of the base body 21'.

### (Fixing of fixed chip to stent graft)

In the fixed chip 20, the forward end portion of the stent graft holder 14 is forced into the lumen 24 (Fig. (11B)) of proximal end portion 23 of the base body 21 and the lumen 25 of the cap 22, and the forward end portion of the above stent graft holder 14 passes through these lumens 24 and 25, whereby the fixed chip 20 is attached to the forward end of the stent graft holder and the cap 22 also is fixed so that it does not come off the base body 21 of the fixed chip.

In the fixed chip 22 in this invention, as shown in Fig. 12(B), the hook holding groove 26 is formed extending from the upper surface (front surface) of the fixed chip 20 and in the center direction nearly perpendicular to a tangent line on the outer circumference (circumference) of the fixed chip 20. Due to the above structure, when the stent graft 60 is housed in the sheath 30, it is converged in the base portion of the hook 66, so that the stent graft 60 in no case comes off the fixed chip 20.
As will be described later, when the release of the stent graft 60 is completed, the tension of the wire does not work any longer, and the base portion of the hook 66 expands together, so that the hook 66 can be easily removed from the fixed chip 20.

Since the fixed chip 20 in this invention is composed of at least two parts (base body 21 and cap 22) in combination, the hook holding groove 26 can be constituted three-dimensionally and stably in form.
A conventional fixed chip having a one-piece structure is produced by a non-molding, one-by-one method in which rods having an outer diameter nearly equivalent to that of a dilator having a guide wire GW lumen have been processed by melt-processing or grinding. In contrast thereto, the fixed chip in this invention can be produced much more easily by injection molding.

### (Assembly of indwelling device and loading of stent graft)

One example of the method of assembling (producing) the stent graft indwelling device of this invention will be explained below.
(1) As already described, the wire 40 is passed through the stent graft 60 as shown in Fig. 8, and the wire 40 is fixed as explained in the above sections [0082] to [0084].

(2) The dilator 10 having the base body portion 11 and the stent graft holder 14 is inserted into the sheath 30 in advance. In this case, the dilator 10 may be inserted in a manner in which the proximal end portion of the base portion 11 enters the opening portion positioned in the forward end of the sheath 30 (enlarged diameter portion 39), or the dilator may be inserted in a manner in which the stent graft holder 14 enters the proximal end portion of the sheath 30.

(3) The proximal end portion (an opposite side to the insertion direction toward a patient) of the stent graft 60 shown in Fig. 4 is diameter-decreased or contracted by means of a dedicated folding tool or device, then inserted, inch-by-inch, with great care not to entail twist, into the sheath 30 through the leading end (enlarged diameter portion 39) of the sheath 30 and fitted to the stent graft holder 14 pre-inserted into the sheath.

(4) When the insertion of the stent graft 60 into the sheath 30 is completed, the hook portion 66 forming the forward end thereof is hooked to or into the hook holding groove 26 of the fixed chip 20, and then the fixed chip 20 is fixed with the leading end of the sheath 30 (enlarged diameter portion 39) (leading end of the stent graft holder 14) to complete the loading of the stent graft.

(5) The stent graft indwelling device in which the stent graft 60, the fixed chip 20 and the wire 40 are loaded or fixed is encased in a proper packaging material and sterilized by a sterilization method such as ethylene oxide gas sterilization, etc. In the above manner, the preparation for use of the stent graft indwelling device is completed.

### (Insertion and indwelling of stent graft and withdrawal of wire)

Figs. 14 to 19 are schematic drawings of one embodiment in which the indwelling device of this invention is inserted into a diseased part (blood vessel) and a stent graft is placed there.
As shown in Fig. 14, the sheath 30 loaded with the stent graft 60 is delivered or transported to a target site along the guide wire GW that is pre-inserted as a guide or rail up to a diseased part (blood vessel) where the stent graft 60 is to be placed, and it is inserted in the diseased part (blood vessel). As shown in Figs. 2 and 3, the guide wire GW is inserted through the guide wire lumen 101 that passes through the center of the base body portion 11 of the dilator.

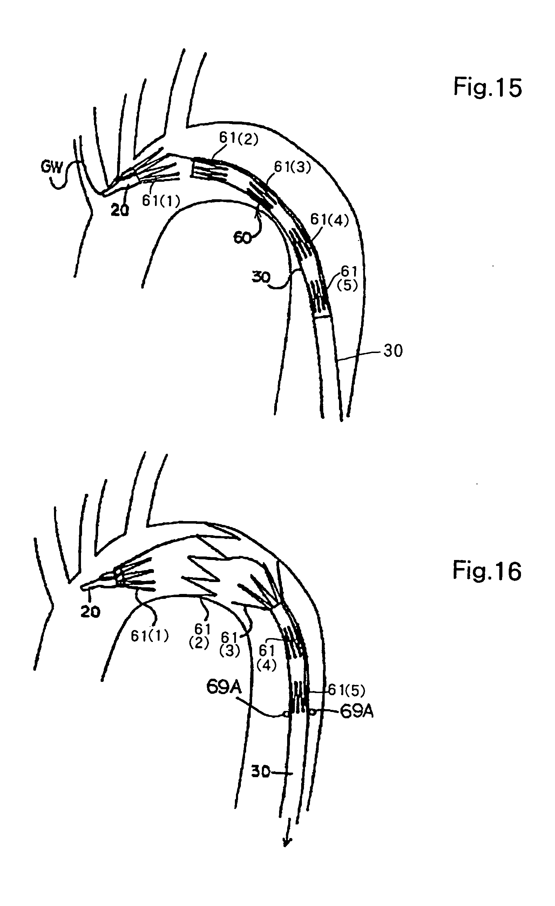

In this state, the sheath 30 is drawn backward (direction opposite to the diseased part) and while the stent graft 60 is pushed outward (Fig 15), the first tubular unit 61(61(1)) inside the diseased part (blood vessel) is expanded.
As shown in Figs. 15 and 16, the sheath 30 is further drawn backward (direction opposite to the diseased part) to release the second, third, ..., tubular units 61 (61(2), 61(3) ...) inside the diseased part (blood vessel), and the positioning thereof is made.

In the curved portion of a blood vessel, the curvature of the stent graft 60 can be adjusted so as to enhance the curvature since the forces of a blood flow and blood pressure exert thereon.
In a state where the stent graft 60 is loaded in the sheath 30, no tension works on the wire 40 for adjusting the indwelling site and insertion angle of the stent graft. And, the stent graft 60 is in a state where it is contracted and folded inside the sheath 30.

The wire 40 is first passed through the holding ring 67 that is one of two holding rings of the hook 66 on the forward end of the stent graft, then passed through the holding ring 69 on the forward end of the first tubular member 61 and wounded around it, and further passed through the other holding ring 68 of the hook 66 and finally passed through the two holding rings 69A and 69A of the rearmost tubular member 61. As described above, therefore, when the stent graft 60 is released at a diseased site, the end of the wire 40 is fixed between the stent graft 60 and the sheath 30 as shown in Fig. 8(A), so that tension is now on the wire when the sheath 30 is drawn backward.

More specifically, when the sheath 30 is drawn backward, the wire 40 is extended (tensioned) using the holding rings 67 and 68 formed in the hook portion 66 as fulcrums, thereby pulling the fixed chip 20 on the forward end of the stent graft 60 toward a smaller curved side (for example, see Fig. 14), and that, when the stent graft 60 is released to the diseased part from the sheath 30, the stent graft 60 can be so tensioned as to increase its curvature.

As shown in Fig. 16, therefore, the forward end of the stent graft 60 is released not in the state of full expansion (for example, when the stent graft has a diameter of 34 mm, the first tubular unit 61(1) (first one) is half opened with a diameter of approximately 20 mm), and even when the remaining second, third ... tubular units 61(2) and 61(3) (second one and third one) are released in a blood vessel, it does not immediately occur that the first tubular unit 61 is completely expanded and fixed in the blood vessel. Therefore, the adjustment of the indwelling position and insertion angle of the stent graft 60 can be easily made by adjusting the position of the sheath 30 and the tension of the wire 40.

As shown in Fig. 17, further, the rearmost tubular unit 61(5) is fixed to the wire 40 with the holding ring 69A, so that the adjustment of the release and indwelling of the stent graft 60 in a blood vessel can be easily performed when the indwelling procedure of the stent graft 60 is made in the blood vessel.
With respect to the determination or manipulation of the position of the inserted stent graft 60, the indwelling position and insertion angle of the stent graft 60 is adjusted by monitoring the X-ray image of the fixed chip 20 containing a contrast medium with an X-ray fluoroscope machine, and while the final adjustment of the angle and indwelling position at which the stent graft 60 can be accurately inserted are made, the third tubular unit 61(3) and the remaining tubular units ... are released. Fig. 18 shows a state where all the tubular units 61 (61(1), 61(2), 61(3), 61(4) and 61(5)) of the stent graft 60 have been released and indwelled in the above manner.

When the entire stent graft is released from the sheath 30 in the above manner, the tension is no longer exerted on the wire 40, so that the hook portion 60 of the stent graft 60 is released from the hook holding groove 26 of the fixed chip 20, thereby completing the indwelling procedure of the stent graft 60 as shown in Fig. 18.

After completion of indwelling of the stent graft 60, an operator of the indwelling device draws the wire end portion 41(42) of the wire 40 through the wire insertion inlet 82 near at hand, whereby the wire 40 can be withdrawn from the stent graft indwelling device.
In the indwelling device of this invention, the wire 40 is passed symmetrically through the holding rings 67, 68, 69 and 69A only, one end portion 41(42) and the other end portion 42(41) of the wire 40 are fixed as explained in the above sections [0082] to [0084]. Thus the whole wire 40 can be easily withdrawn from the indwelling device in the above manner after the indwelling of the stent graft 60 is completed.

### (Adjustment of fixing position of stent graft 60 in celiac arterial trunk branched from aorta thoracica)

One example of a method of fixing (or indwelling) the stent graft 60 in a celiac artery with the fixed chip 20' free of a cap shown in Fig. 13 will be explained with reference to Figs. 19 to 22. First, when Figs. 19(A) to 19(C) are referred to, an indwelling device is assembled in the same manner as in the above explanation except that the hook 66 of the stent graft 60 is attached to the hook-attaching groove 26' without causing it to engage with the fixed chip 20', and the indwelling device is loaded with the stent graft 60 as shown in Fig. 19(B).

As shown in Fig. 19(C), the rearmost tubular member 61 of the stent graft 60 is folded into the sheath 30, and when the largest diameter of backward end of the housed tubular member 61 (in a state where it is compressed to decrease its diameter) is taken as S1, and when the largest diameter of forward end of the base portion 11 of the dilator 10 loaded in the sheath 30 is taken as S2, it is preferred to attain S2>S1.

For example, the backward end of the tubular member 61 can be decreased in diameter by fastening or tying up the outer circumference of the backward end of the tubular member 61 with a wire (not shown) and compressing it.
When prepared in the manner as described above, it is facilitated that the stent graft 60 can be easily fixed in a predetermined site of a branched celiac artery by pushing it with the forward end of the dilator base portion 11 to push it out of the sheath 30.

As shown in Fig. 19(A), the sheath 30 loaded with the stent graft 60 is delivered to a targeted site or position along, and using as a guide, the guide wire GW (not shown) that is pre-inserted into a diseased part (blood vessel) where the stent graft is to be indwelled, and it is inserted in the diseased part (blood vessel).

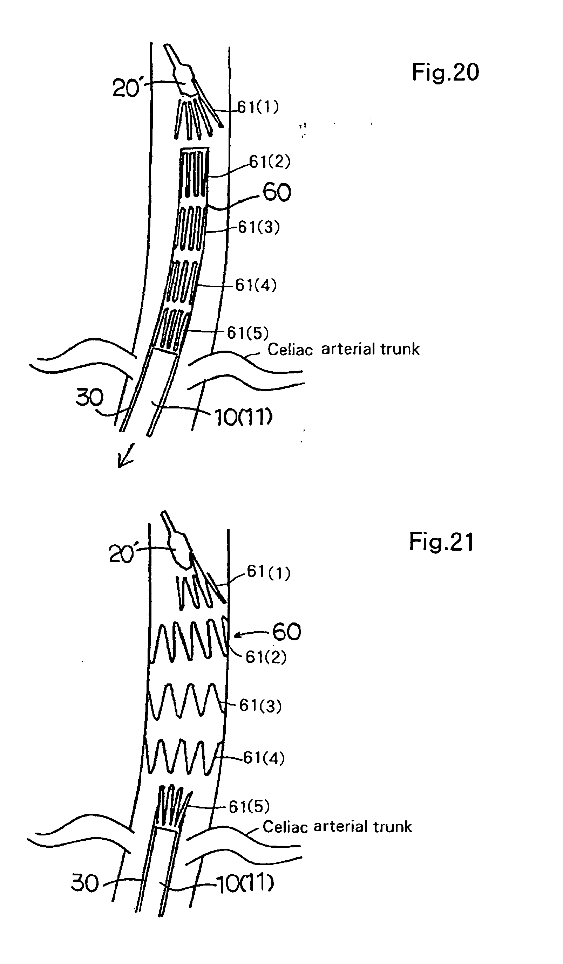

As shown in Fig. 20, the backward end of the stent graft 60 is pushed with the forward end of the dilator base body 11 while the sheath 30 is pulled backward (opposite to the diseased part), thereby the first tubular unit 61(1) is pushed out and expanded in the diseased part (blood vessel).
As shown in Fig. 21, further, the sheath 30 is pulled backward (opposite to the diseased part), and the second, third, ... tubular units 61(2), 62(3) ... are released in the diseased part (blood vessel) and positioned.

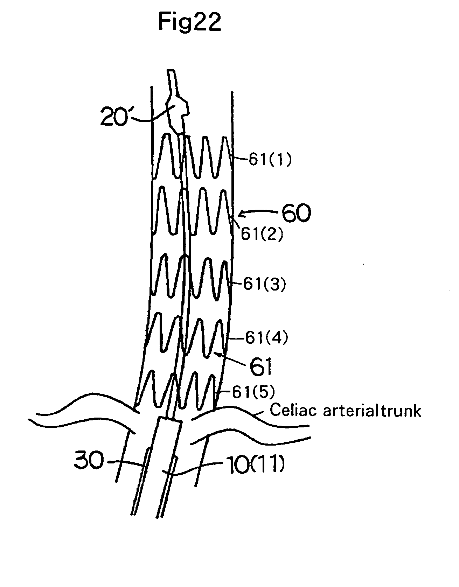

Fig. 22 shows a state where all the tubular units 61(1), 61(2), 61(3), 61(4) and 61(5) have been released and the final positioning of the tubular member 61 has been made so that the backward end of the tubular member 61 is arranged to be positioned at the celiac artery.

### Industrial Applicability

(1) In this invention, when the stent graft is indwelled in a blood vessel, a plurality of holding rings of a wire can be attached to the backward end of the rearmost tubular unit of the stent graft as a means of adjusting the insertion angle and/or indwelling position of the stent graft, thereby making the release and indwelling of the stent graft in the blood vessel easy.

(2) In this invention, when the maneuver procedure of fixing the stent graft (adjustment of the insertion angle and the indwelling position) is performed, there can be employed a fixed chip having a base body and a cap, containing different amounts of a contrast medium as means of adjusting the insertion angle and/or indwelling position of the stent graft, so that the base body and the cap are clearly distinguishable in contrast during imaging procedure, and the position of the hook (of the stent graft) engaging with the hook holding groove can be clearly monitored. Thus, the maneuver procedure of fixing the stent graft (adjustment of the insertion angle and the indwelling position) is made easy.

(3) In this invention, the reinforcement tube is provided on the inner circumference of the dilator, and a hard material and a soft material are alternately arranged from the leading end portion to the proximal end portion of the above reinforcement tube, so that there can be produced a high curvature-following capability to a blood vessel even when the blood vessel is curved and irregular.

(4) In this invention, the wire lumen for passing the wire through is formed in a cross section of the base body of the dilator from the proximal end to the leading end, so that easy remove of the wire from the dilator can be made.

(5) In this invention, the forward end of the sheath is formed in a curved form, the above forward end and the curvature radius R thereof are formed to satisfy any of the followings: (A) "type A", a curvature radius R: 140 - 170, (B) "type J", a curvature radius R: 45 - 90, (C) "type LU", a curvature radius R: 60 - 70, or (D) "type U", a curvature radius R: 60 - 70. Therefore, the sheath can be easily inserted even into an irregular blood vessel, with an improved curve-following capability to the blood vessel.

(6) In this invention, at least one opening portion is formed in the tubular member covering the stent graft, and it is thereby ensured that blood easily flows in small blood vessels (brachiocephalic arterial trunk, left common carotid artery and left subclavian artery) branched from aorta thoracica.

(7) In this invention, the stent graft is formed by covering the stent, and in a state where the tubular member in the backmost position of the stent graft is folded and housed in the sheath, it is adjusted to ensure that the largest diameter S1 of the backward end of the rearmost tubular member is smaller than the largest diameter S2 of the leading end of the dilator loaded in the sheath, the adjustment of the stent graft to the fixing position in celiac artery branched from aorta thoracica can be accurately performed.

## Claims

1. A stent graft indwelling device (1) having a dilator (10) having a stent graft holder (14) and a sheath (30) loadable with a stent graft (60) held on the stent graft holding portion (14) of said dilator (10),
Said stent graft (6) being formed of a plurality of connected tubular units (61), the stent graft indwelling device having means of adjusting the insertion angle and/or indwelling position of said stent graft when said stent graft is released from said sheath (30) for indwelling,
the means of adjusting the insertion angle and/or indwelling position of said stent graft (6) comprising a fixed chip (20) containing a contrast medium and/or a holding ring (69A) of a wire 40 attached to the rearmost one of said tubular unit (61) of said stent graft (60).

2. The stent graft indwelling device (1) of claim 1, which has the dilator (10) having a fixed chip (20) in its forward end and a sheath (30) loadable with the stent graft (60) held on the stent graft holder (14) of said dilator (10),
the stent graft indwelling device (1) being adapted to reduce impairment of a blood vessel and disengagement of an embolic substance from a blood vessel wall by flexing the stent graft (60) by harnessing the expansion power of the stent graft (60), thereby making the fixed chip (20) free from the blood vessel wall, while the stent graft (60) is released from the stent graft indwelling device (1) for indwelling.

3. The stent graft indwelling device (1) of claim 1 or 2, wherein said dilator (10) has a base body portion (11), the stent graft holder (14) and the fixed chip (20),
said dilator (10) has a through hole and a reinforcement tube (13) being attached to an inner circumference thereof,
said reinforcement tube (13) is formed of a hard material (13A), a soft material (13B) and a hard material (13A) that are alternately arranged from a leading end portion to a proximal end portion.

4. The stent graft indwelling device of any one of claims 1 to 3, wherein a wire lumen (100) for letting through a wire (40) to be used for adjusting the indwelling position and/or insertion angle of the stent graft (60) is formed in cross section of said dilator from the proximal end to the leading end of said dilator (10).

5. The stent graft indwelling device of any one of claims 1 to 4, wherein the proximal end portion (12) of said dilator is provided with a fixing means for the wire (4) that is used for adjusting the indwelling position and/or insertion angle of the stent graft (60).

6. The stent graft indwelling device (1) of any one of claims 1 to 5, wherein said sheath (3) has a forward end formed in the form of a curvature, and the forward end curvature form and a curvature radius R being selected from
(A) [type A] and a curvature radius R of 140 to 170,
(B) [type J] and a curvature radius R of 45 to 90,
(C) [type LU] and a curvature radius R of 60 to 70, and
(D) [type U] and a curvature radius R of 60 to 70.

7. The stent graft indwelling device (1) of any one of claims 1 to 6, wherein said stent graft (60) is formed of a plurality of tubular units (61) connected in the center axis direction thereof, a holding ring (69) and a hook portion (66) are attached to an outer circumference of the first one of the tubular units (61), holding rings (67, 68) are attached to said hook portion (66) and a plurality of holding rings (69A) are attached to a rear portion of the rearmost one of the tubular units (61).

8. The stent graft indwelling device of any one of claims 1 to 7, wherein said stent graft (60) is covered with a tubular member (65) and at least one opening portion (65A) is formed in said tubular member (65).

9. The stent graft indwelling device of any one of claims 1 to 8, wherein said fixed chip (20) is composed of a base body (21) and a cap (22), a hook holding groove (26) is formed between said cap (22) and an upper opening portion of the base body (21), and the base body and the cap contain contrast medium in different amounts.

10. The stent graft indwelling device (1) of any one of claims 1 to 9, wherein said wire (40) extends from an inside of the stent graft (6) to a wire fixing means formed in the proximal end portion (12) of the dilator through a lumen (100) formed from the base body portion (11) of the dilator (10) to the base portion (12) of the dilator and fixed, and, when the stent graft (60) is released from the sheath (30), said wire is capable of adjusting the release angle and indwelling position of the stent graft (60) by applying tension to compress the stent graft (60) with the wire (40) during the release of the stent graft (60).

11. The stent graft indwelling device (1) of any one of claims 1 to 10, wherein a wire end portion (41) fixed to said fixing means is loosened and pulled out of the holding rings (67, 68, 69 and 69A) of said stent graft (60), whereby the wire can be pulled out of said stent graft indwelling device (1).

12. The stent graft indwelling device (1) of any one of claims 1 to 11, wherein a forward end of the stent graft holder (14) is forced into a lumen (24) of proximal end portion (23) of the base body (21) and a lumen (25) of the cap (22), and the forward end portion of the above stent graft holder (14) passes through these lumens (24) and (25) to fix the cap (22) and the base body (21).

13. The stent graft indwelling device (1) of any one of claims 1 to 12, wherein said means for adjusting the insertion angle and/or indwelling position of said stent graft (60) includes the wire fixing means attached to the base body portion (11) of the dilator (10), the stent graft holder (14) and the fixed chip (20) and the proximal end portion (12) of the dilator; a hook portion (66) formed in the leading end of the first tubular unit (61) of the stent graft (60), holding rings (67 and 68) formed in said hook portion (66), a plurality of holding rings (69A) formed in the front portion of the first tubular unit (61), a plurality of holding rings (69A) formed in the back portion of the tubular unit (61) forming the backmost row; and the wire (40) that passes through a wire lumen (100) formed between the holding rings (67,68,69,69A) and the dilator (10) and the sheath (30) or from the leading end (10) of the dilator (10) to the trailing end (12) of the dilator and a wire insertion inlet (82) and fixed with said wire fixing means.

14. A stent graft (60) loadable in the dilator (10) of said stent graft indwelling device (1) recited in any one of claims 1 to 13, which is constituted of a plurality of tubular units (61) connected in the central axis direction thereof, a holding ring (69) and a hook portion (66) being attached to an outer circumference of a first one of the tubular units (61), and holding rings (67 and 68) being attached to said hook portion (66),
a plurality of holding rings (69A) being attached to a back of the rearmost one of said plurality of the tubular units (61).

15. A fixed chip (20) that is to be attached to the dilator (10) of the stent graft indwelling device (1) recited in any one of claims 1 to 13 and formed of a base body (21) and a cap (22), a hook holding groove (26) being formed between the cap (22) and an upper opening portion of the base body (21), the base body (21) and the cap (22) containing a contrast medium in different amounts.

16. A stent graft indwelling device (1') having a dilator (10) having the fixed chip (20') and a sheath (30) loadable with a stent graft (60) held on a stent graft holder (14) of said dilator (10), wherein in a state where the rearmost tubular member (61) of the stent graft (60) is folded into the sheath (30), the largest diameter S1 of backward end of the housed tubular member (61) is smaller than the largest diameter S2 of forward end of the base body portion (11) of the dilator (10) loaded in the sheath (30).
